# EUROPEAN PATENT SPECIFICATION

(11) **EP 3 476 437 B1**
(45) Date of publication and mention of the grant of the patent: **12.08.2020**
(21) Application number: 18201293.0
(22) Date of filing: 18.10.2018
(51) Int. Cl.: A61N 7/02, A61B 17/56, A61B 90/14, A61B 18/00

(54) **DEVICE FOR THERMAL ABLATION ON THE KNEE JOINT WITH HIGH INTENSITY FOCUSED ULTRA SOUND**
VORRICHTUNG ZUR THERMISCHEN ABLATION AM KNIEGELENK MIT HOCHINTENSIVEM FOKUSSIERTEM ULTRASCHALL
DISPOSITIF D'ABLATION THERMIQUE SUR L'ARTICULATION DU GENOU PAR ULTRASONS FOCALISÉS DE HAUTE INTENSITÉ

(30) Priority: 18.10.2017 EP 17197059
(43) Date of publication of application: 01.05.2019
(73) Proprietor: Johann Wolfgang Goethe-Universität Frankfurt, 60323 Frankfurt am Main (DE)
(72) Inventor: Korkusuz, Hüdayi, 60528 Frankfurt am Main (DE); Klingebiel, Felix, 60329 Frankfurt am Main (DE)
(74) Representative: Lucke, Andreas

(56) References cited:
- EP-A1- 2 570 083
- EP-A2- 1 917 950
- WO-A1-2017/089457
- GB-A- 2 098 484
- US-A1- 2004 199 072
- US-A1- 2012 234 329
- KAROLY FOLDES ET AL: "Magnetic Resonance Imaging-guided Focused Ultrasound Synovectomy", SCANDINAVIAN JOURNAL OF RHEUMATOLOGY, ALMQVIST & WIKSELL PERIODICAL CO., STOCKHOLM, SE, vol. 28, no. 4, 12 January 1999 (1999-01-12), pages 233-237, XP009511969, ISSN: 0300-9742, DOI: 10.1080/03009749950155607

## Description

### Background of the invention

As described in several publications the HIFU (High intensity focused ultrasound) treatment is able to offer a new approach in the field of thermal ablation and noninvasive destruction of human tissue. This technique is used in several medical fields such as a treatment for the fibro adenoma of the breast [1] and thyroid ablation [2]. But since now the full potential of this technique is not fully discovered and our approach was to test the usage of the HIFU for the thermal ablation as a noninvasive treatment for the synovitis of the human knee joint, as the so called "Thermal Synovectomia". The primary question for our experiment was, whether a thermal effect can be performed on the target tissue in the knee joint, the synovial stratum. Our secondary questioning was whether one can improve the intended therapy by fixation of the knee and compressing the joint capsule in aspects of maximizing the distance between the two potentially pain causing areas during the thermal ablation; the skin and the periosteal.

The viability of MRI-guided high power focused ultra sound (FUS) to perform synovectomy non-invasively was studied in Karoly Foldes et al., "Magnetic Resonance Imaging-guided Focused Ultrasound Synovectomy" in Scandinavian Journal of Rheumatology, 12 January 1999, Scandinavian University Press.

### Summary of the invention

According to the present invention the problem is solved by a device for application in thermal ablation with high-intensity focused ultra sound medical device on a knee joint of a patient according to claim 1.

Preferred embodiments of the invention are defined in the appended dependent claims.

### Description of the preferred embodiments of the invention

Before the present invention is described in more detail below, it is to be understood that this invention is not limited to the particular methodology, protocols and devices described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Amounts and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "130° to 150°" should be interpreted to include not only the explicitly recited values of 130 to 150, but also include individual values and sub-ranges within the indicated range. Thus, included in this numerical range are individual values such as 132, 133.5, 134, ..., 149, 150 and sub-ranges such as from 133 to 142, from 135 to 144,and from 140 to 150, etc. This same principle applies to reciting only one numerical value, such as "at least 10%". Furthermore, such an interpretation should apply regardless of the breadth of the range or the characteristics being described.

### Method

The method according to an example not belonging to the claimed invention as defined in the claims but useful for the understanding thereof is a method to perform thermal ablation with high-intensity focused ultra sound on the knee joint of a patient, comprising:
a) fixating the knee joint, by a device that limits a movement of the knee joint, wherein the fixation immobilizes the knee joint; and/or
b) compressing the knee, by a device with a means for compressing both lateral sides of the knee, wherein the compression is located and constrained to both lateral sides of the knee.

Preferably, the method further comprises:
applying thermal ablation to the knee joint, by a high-intensity focused ultra sound medical device, wherein the thermal ablation is aimed at and constraint to the knee joint.

Preferably, the method further comprises:
applying thermal ablation to the synovia located in the knee joint, by a high-intensity focused ultra sound medical device, wherein the thermal ablation is aimed at and constraint to the synovia located in the knee joint.

Preferably, the method further comprises:
a) fixating the synovia of the knee, by a device with a means for limiting the movement of the synovia of the knee, wherein the device fixates the synovia of the knee; and/or
b) compressing the synovia of the knee, by a device with a means for compressing the synovia of the knee, wherein the device locates and constrains the compression means to the synovia of the knee.

Preferably, the method further comprises:
a) compressing the knee above the knee joint in the region of the distal femoral metaphysis, by a device with a means for compressing both lateral sides of the knee above the knee joint in the region of the distal femoral metaphysis, wherein the device locates and constrains the compression means to both lateral sides of the knee above the knee joint in the region of the distal femoral metaphysis; and/or
b) compressing the knee below the knee joint in the region of the proximal tibial and fibular metaphysis, by a device with a means for compressing both lateral sides of the knee below the knee joint in the region of the proximal tibial and fibular metaphysis, wherein the device locates and constrains the compression means to both lateral sides of the knee below the knee joint in the region of the proximal tibial and fibular metaphysis.

Preferably, the method further comprises:
applying a pressure to increase the distance between the skin and periosteum of the knee by at least 10% and/or synovia and periosteum of the knee by at least 40%, by a device with a means for applying a pressure to increase the distance between the skin and periosteum of the knee by at least 10% and/or synovia and periosteum of the knee by at least 40%, wherein the device locates and constrains the distance increment to the region between the skin and periosteum of the knee and/or synovia and periosteum of the knee.

Preferably, the method further comprises:
fixating the knee joint at a fixed angle in the range of 130° to 150°, by a device with a means for limiting the movement of the knee joint, wherein the device fixates and immobilizes the knee joint at a fixed angle in the range of 130° to 150°.

### Device

The device of the present invention is a device for application in thermal ablation with high-intensity focused ultra sound medical device on a knee joint of a patient, comprising:
a) a means for limiting the movement of the knee joint, wherein the device immobilizes the knee joint; and/or
b) a means for compressing both lateral sides of the knee, wherein the device locates the compression means to both lateral sides of the knee.

The device further comprises:
a means for applying a pressure to increase the distance between the skin and periosteum of the knee by at least 10% and/or synovia and periosteum of the knee by at least 40%, wherein the device locates and constrains the distance increment to the region between the skin and periosteum of the knee and/or synovia and periosteum of the knee.

Preferably, the device further comprises:
a) a means for limiting the movement of the synovia of the knee, wherein the device fixates the synovia of the knee; and/or
b) a means for compressing the synovia of the knee, wherein the device locates and constrains the compression means to the synovia of the knee.

Preferably, the device further comprises:
a) a means for compressing both lateral sides of the knee above the knee joint in the region of the distal femoral metaphysis, wherein the device locates and constrains the compression means to both lateral sides of the knee above the knee joint in the region of the distal femoral metaphysis; and/or
b) a means for compressing both lateral sides of the knee below the knee joint in the region of the proximal tibial and fibular metaphysis, wherein the device locates and constrains the compression means to both lateral sides of the knee below the knee joint in the region of the proximal tibial and fibular metaphysis.

Preferably, the device further comprises:
a means for limiting the movement of the knee joint, wherein the device fixates and immobilizes the knee joint at a fixed angle in the range of 130° to 150°.

More preferably, said device, wherein:
a) the means for compression from both lateral sides is operated with equal force on both lateral sides; and/or
b) the force of the means for compression is adjusted by one or multiple screws or knobs; and/or
c) the means for compression consist of concave pads; and/or
d) the means for compression consist of a material which is easy to clean.

More preferably, said device, wherein:
the device is topless, such that the entire or partial skin enclosing the patella and/or the entire or partial skin above the patella and/or the entire or partial skin below the patella is not covered by the device.

More preferably, said device, wherein:
a) the device is firmly attached to a solid surface, such as a stretcher, hospital bed or divan bed, wherein a rigid connection between the device and the solid surface persists; and/or
b) the device is firmly attached to a high-intensity focused ultra sound medical device used for thermal ablation, wherein a rigid connection between the device and the high-intensity focused ultra sound medical device persists.

### Brief description of the figures

**Figure 1** Thermal ablation performed on the uncompressed knee shown for the transverse section of the knee.
**Figure 2** Thermal ablation performed on the compressed knee shown for the transverse section of the knee.

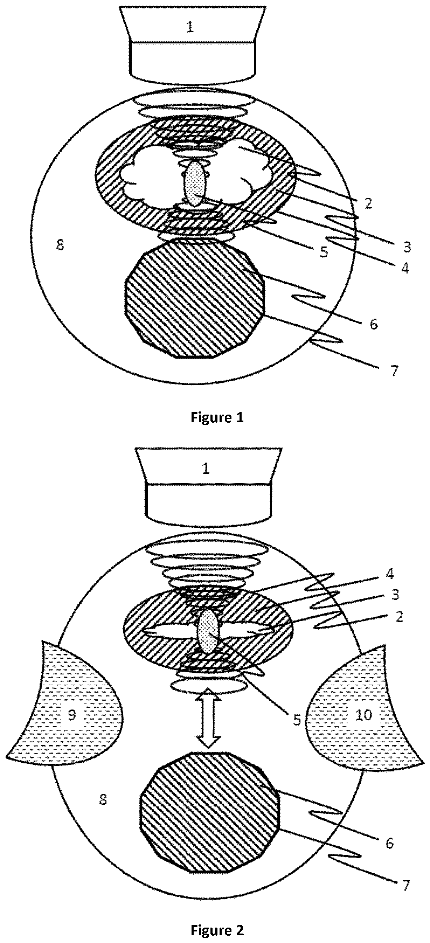

**Figure 2** Thermal ablation performed on the compressed knee shown for the longitudinal section of the knee.

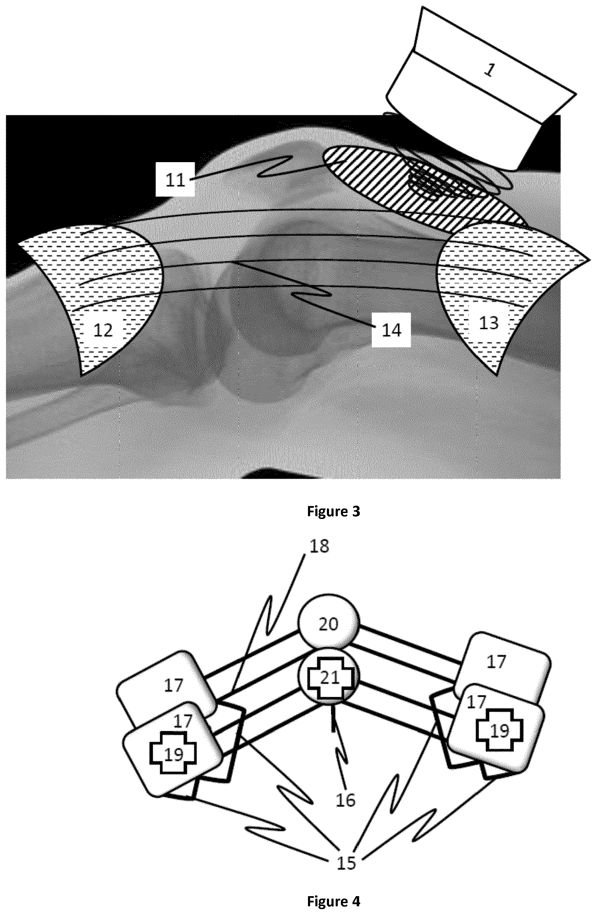

**Figure 4** Device for fixation and compression of the knee.

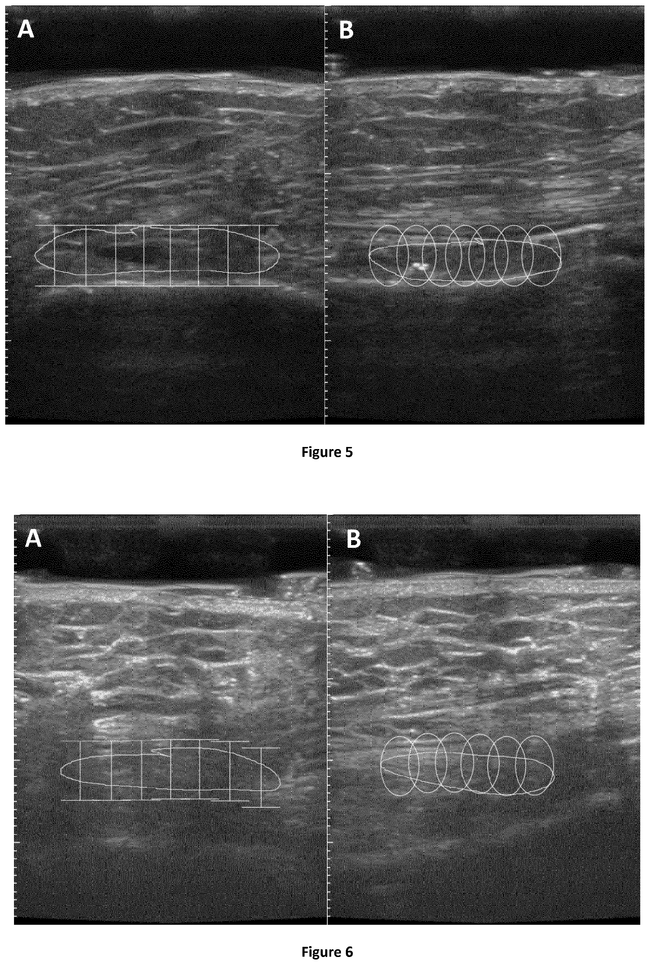

**Figure 5a** Antiradial view of knee 1 with targeted area not compressed.
**Figure 5b** Radial view of knee 1 with targeted area not compressed.
**Figure 6a** Antiradial view of knee 1 with targeted area compressed.
**Figure 6b** Radial view of knee 1 with targeted area compressed.

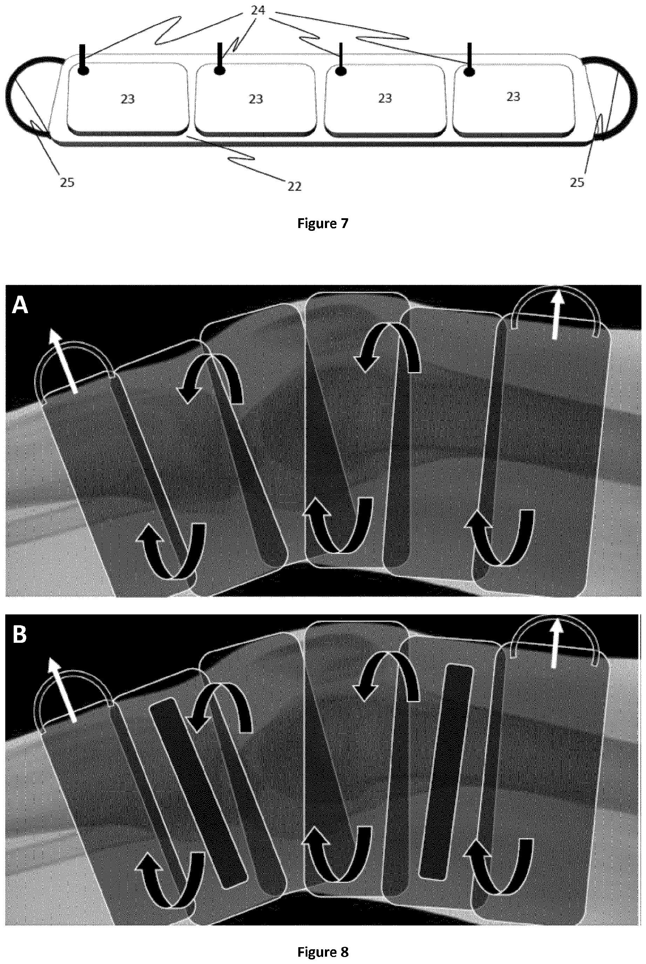

**Figure 7** Device for fixation and compression of the knee.
**Figure 8a** circular winding of the vacuum bandage without compression pads
**Figure 8b** circular winding of the vacuum bandage with compression pads

### Examples

### Methods

Trials have been performed on three knee joints of very recent body donors, which have been opened before but have been closed with surgical stitches. After a primary ultrasonic displaying of the synovial stratum, the HIFU transducer has been placed vertically above the knee joint and focusing the synovial stratum. With each of the knee joints we performed 2 trials; one with fixation of the knee joint and compression of the joint capsule and one without any compression or fixation. At each trial 7-10 pulses of the HIFU were performed at the equal amount of areas. The transmitted energy per pulse ranged from 416,1 J/area up to 528,2 J/area and the power of each pulse has been in the range between 54,8 W/area and 77,2 W/area. After the knee has undergone both trials the skin has been checked for not intended signs of burns and then has been opened up to the bone immediately to check for thermal effects and potentially thermal injuries at non-targeted tissues and the bone.

The fixation of the knee was performed by two C-ferrules placed lateral below and above the joint capsule so that the entire knee joint including the synovia has been compressed and increased the distance between the targeted area, the skin and the periosteal for maximizing the thermal effect on a more compact tissue.

**Table 1 shows the specifications of the performed treatment.**

| Knee / status | Amount of pulses | Transmitted Energy J/area | Transmitted power W/area |
|---|---|---|---|
| Knee 1 / not compressed | 7 | 528,2 J/Area | 77,2 W/Area |
| Knee 1 / compressed | 9 | 416,1 J/Area | 54,8 W/Area |
| Knee 2 / not compressed | 8 | 498,1 J/Area | 68,9 W/Area |
| Knee 2 / compressed | 10 | 494,1 J/Area | 64,7 W/Area |
| Knee 3 / not compressed | 9 | 491,0 J/Area | 64,7 W/Area |
| Knee 3 / compressed | 9 | 435,2 J/Area | 57,4 W/Area |

### Results

Neither the skin nor the periosteal/bone have shown signs of being thermally affected or injured by the procedure. The manual inspection of the targeted synovial stratum in the opened up knee joint, after every knee has undergone the trial with and without compression, has shown a thermal effect at the targeted tissue, which was able to be detected and felt by hand as a raise in temperature.

The amount of tissue, which was targeted with each pulse of the HIFU, was 0,084 cm³ to 0,085 cm³.

By the kind of compression, we used, the distance between the tender skin and periosteal has been increased by 20%, 35,56% and 50,78% which depended on the overall size of the leg and knee joint. Especially the distance between the targeted area, the synovial stratum and the periosteal has been seized up to 57,5%, 49,83% and 87,4%.

The trials have shown that it is possible to perform a thermal effect at the targeted synovial stratum by using the HIFU. The possibility for using the HIFU for treating the synovitis in the knee joint in a noninvasive way is proven and needs to be examined further to its full potential. As well it seems better to compress the joint capsule so that the targeted synovial stratum is more compact and the heat up area per pulse with 0,084 cm³ is used more efficiently and at the same time one can assume that the heat stays inside of the tissue longer, the more compressed it is. This aspect could not be completely clarified by the trials because the knee joints of the body donors have been opened before, therefore there has been air in the joint capsule and the tissue, which wouldn't have been there naturally, which certainly lowers the efficiency of the performed trials. Therefore, one can assume that the thermal effect, used upon a living patient, would be more efficient. In view of the treatment of a patient the lateral compression of the joint capsule of the knee would have the positive effect of lowering the potential level of pain because the treated area lies within a greater distance from the two tender structures, the skin and the periosteal which also decreases the possibility of thermal injuries regarding them.

**Table 2 shows relevant parameters accumulated during the treatment.**

| Knee/ Status | Treated area (cm³) | Treated area per pulse (cm³) | Distance skin/ periosteal (mm) | Raise of distance (skin/periosteal) by compression (mm/%) | Distance target area/ periosteal (mm) | Raise of distance target area/periosteal (mm/%) |
|---|---|---|---|---|---|---|
| Knee 1 / not compressed | 0,95 cm³ | 0,084 cm³ | 22,8 mm | 4,56 mm / 20% | 4,73 mm | 2,72 mm / 57,5% |
| Knee 1 / compressed | 0,76 cm³ | 0,085 cm³ | 27,36 mm | | 7,45 mm | |
| Knee 2 / not compressed | 0,68 cm³ | 0,084 cm³ | 17,94 mm | 6,38 mm / 35,56 % | 5,78 mm | 2,88 mm / 49,83 % |
| Knee 2 / compressed | 0,84 cm³ | 0,084 cm³ | 24,32 mm | | 8,66 mm | |
| Knee 3 / not compressed | 0,76 cm³ | 0,084 cm³ | 21,29 mm | 10,81 mm / 50,78% | 7,3 mm | 6,38 mm / 87,4 % |
| Knee 3 / compressed | 0,76 cm³ | 0,084 cm³ | 32,1 mm | | 13,68 mm | |

### List of reference signs

- 1: HIFU medical device
- 2: Synovial liquid
- 3: Synovial membrane
- 4: Joint capsule
- 5: Treated volume;
- 6: Femur
- 7: Periost
- 8: Knee
- 9: Left compression pad
- 10: Right compression pad
- 11: Synovia
- 12: Lower compression pad
- 13: Upper compression pad
- 14: Connecting structure
- 15: Clamps
- 16: Connections of adjustment screws
- 17: Compression pad
- 18: Connecting structure
- 19: Compression means
- 20: Pad
- 21: Angle adjustment means
- 22: Vacuum bandage
- 23: Fillable packet
- 24: Valve
- 25: Handle

### References

[1] Kovatcheva R, Zaletel K, Vlahov J, Stoinov J; 2017; Long-term efficacy of ultrasound-guided high-intensity focused ultrasound treatment of breast fibroadenoma; Journal of Therapeutic Ultrasound; 5:1
[2] Korkusuz H, Sennert M, Fehre N, Happel C, Grünwald F; 2015; Localized Thyroid Tissue Ablation by High Intensity Focused Ultrasound: Volume Reduction, Effects on Thyroid Function and Immune Response; Fortschr Röntgenstr; 187(11): 1011-1015

## Claims

1. A device for application in thermal ablation with high-intensity focused ultra sound medical device on a knee joint of a patient, comprising:
a) a means for limiting the movement of the knee joint, wherein the device immobilizes the knee joint; and/or
b) a means for compressing both lateral sides of the knee, wherein the device locates the compression means to both lateral sides of the knee;
**characterised in that** the device further comprises:
a means for applying a pressure to increase the distance between the skin and
periosteum of the knee by at least 10% and/or synovia and periosteum of the knee by at least 40%, wherein the device locates and constrains the distance increment to the region between the skin and periosteum of the knee and/or synovia and periosteum of the knee.

2. A device according to claim 1, further comprising:
a) a means for limiting the movement of the synovia of the knee, wherein the device fixates the synovia of the knee; and/or
b) a means for compressing the synovia of the knee, wherein the device locates and constrains the compression means to the synovia of the knee.

3. A device according to claim 1, further comprising:
a) a means for compressing both lateral sides of the knee above the knee joint in the region of the distal femoral metaphysis, wherein the device locates and constrains the compression means to both lateral sides of the knee above the knee joint in the region of the distal femoral metaphysis; and/or
b) a means for compressing both lateral sides of the knee below the knee joint in the region of the proximal tibial and fibular metaphysis, wherein the device locates and constrains the compression means to both lateral sides of the knee below the knee joint in the region of the proximal tibial and fibular metaphysis.

4. A device according to claim 1, further comprising:
a means for limiting the movement of the knee joint, wherein the device fixates and
immobilizes the knee joint at a fixed angle in the range of 130° to 150°.

5. A device according to claims 1 to 4, wherein:
a) the means for compression from both lateral sides is operated with equal force on both lateral sides; and/or
b) the force of the means for compression is adjusted by one or multiple screws or knobs; and/or
c) the means for compression consist of concave pads; and/or
d) the means for compression consist of a material which is easy to clean.

6. A device according to claims 1 to 5, wherein:
the device is topless, such that the entire or partial skin enclosing the patella and/or
the entire or partial skin above the patella and/or the entire or partial skin below the patella is not covered by the device.

7. A device according to claims 1 to 6, wherein:
a) the device is firmly attached to a solid surface, such as a stretcher, hospital bed or divan bed, wherein a rigid connection between the device and the solid surface persists; and/or
b) the device is firmly attached to a high-intensity focused ultra sound medical device used for thermal ablation, wherein a rigid connection between the device and the high-intensity focused ultra sound medical device persists.

## Patentansprüche

1. Vorrichtung zur Anwendung bei thermischer Ablation mit einem Hochintensität-Fokussierter-Ultraschall medizinischen Gerät an einem Kniegelenk eines Patienten, die Folgendes umfasst
a) Mittel zur Begrenzung der Bewegungen des Kniegelenks, wobei die Vorrichtung das Kniegelenk immobilisiert; und/oder
b) Mittel zum Komprimieren beider lateraler Seiten des Knies, wobei die Vorrichtung das Mittel zum Komprimieren an beiden lateralen Seiten des Knies anordnet; **dadurch gekennzeichnet, dass** die Vorrichtung ferner Folgendes umfasst:
Mittel zum Ausüben von Druck, um den Abstand zwischen der Haut und dem Periost des Knies um mindestens 10% und/oder zwischen der Synovia und dem Periost des Knies um mindestens 40% zu vergrößern, wobei die Vorrichtung das Abstandsinkrement auf den Bereich zwischen der Haut und dem Periost des Knies und/oder zwischen der Synovia und dem Periosteum des Knies lokalisiert und beschränkt.

2. Vorrichtung nach Anspruch 1, die ferner Folgendes umfasst:
a) Mittel zur Begrenzung der Bewegungen der Synovia des Knies, wobei die Vorrichtung die Synovia des Knies fixiert; und/oder
b) Mittel zum Komprimieren der Synovia des Knies, wobei die Vorrichtung das Kompressionsmittel auf die Synovia des Knies lokalisiert und fixiert.

3. Vorrichtung nach Anspruch 1, die ferner Folgendes umfasst:
a) Mittel zum Komprimieren beider lateraler Seiten des Knies oberhalb des Kniegelenks im Bereich der distalen femoralen Metaphyse, wobei die Vorrichtung die Kompressionsmittel an beiden lateralen Seiten des Knies oberhalb des Kniegelenks im Bereich der distalen femoralen Metaphyse lokalisiert und fixiert; und/oder
b) Mittel zum Komprimieren beider lateraler Seiten des Knies unterhalb des Kniegelenks im Bereich der proximalen tibialen und fibularen Metaphyse, wobei die Vorrichtung das Kompressionsmittel an beiden lateralen Seiten des Knies unterhalb des Kniegelenks im Bereich der proximalen tibialen und fibularen Metaphyse lokalisiert und fixiert.

4. Vorrichtung nach Anspruch 1, die ferner Folgendes umfasst:o Mittel zur Begrenzung der Bewegungen des Kniegelenks, wobei die Vorrichtung das Kniegelenk in einem festen Winkel im Bereich von 130° bis 150° fixiert und immobilisiert.

5. Vorrichtung nach den Ansprüchen 1 bis 4, wobei:
a) die Mittel zur Kompression von beiden lateralen Seiten mit gleicher Kraft auf beiden lateralen Seiten angewendet werden; und/oder
b) die Kraft der Mittel zum Komprimieren durch eine oder mehrere Schrauben oder Knöpfe eingestellt wird; und/oder
c) die Mittel zum Komprimieren aus konkaven Pads bestehen; und/oder
d) die Mittel zum Komprimieren aus einem Material bestehen, das leicht zu reinigen ist.

6. Vorrichtung nach den Ansprüchen 1 bis 5, wobei:
die Vorrichtung Oberteil-frei ist, so dass die Haut, die die Patella ganz oder teilweise umschließt, und/oder die Haut, die sich ganz oder teilweise über der Patella befindet,
und/oder die Haut, die sich ganz oder teilweise unter der Patella befindet, von der Vorrichtung nicht bedeckt wird.

7. Vorrichtung nach den Ansprüchen 1 bis 6, wobei:
a) die Vorrichtung fest mit einer festen Oberfläche, wie einer Trage, einem Krankenhausbett oder einer Liege verbunden ist, wobei eine starre Verbindung zwischen der Vorrichtung und der festen Oberfläche bestehen bleibt; und/oder
b) die Vorrichtung fest mit einem Hochintensität-Fokussierter-Ultraschall medizinischen Gerät zur thermischen Ablation verbunden ist, wobei eine starre Verbindung zwischen dem Gerät und dem Hochintensität-Fokussierter-Ultraschall medizinischen Gerät bestehen bleibt.

## Revendications

1. Dispositif d'ablation thermique avec un dispositif médical à ultrasons concentrés à haute intensité pour l'application sur une articulation de genou d'un patient comprenant :
a) un moyen pour limiter le mouvement de l'articulation du genou, dans lequel le dispositif immobilise l'articulation du genou ; et/ou
b) un moyen pour comprimer les deux côtés latéraux du genou, dans lequel le dispositif positionne le moyen de compression sur les deux côtés latéraux du genou ;
**caractérisé en ce que** le dispositif comprend en outre :
un moyen pour appliquer une pression afin d'augmenter la distance entre la peau et le périoste du genou d'au moins 10% et/ou la synovie et le périoste du genou d'au moins 40%, dans lequel le dispositif positionne et contraint l'incrément de distance à la région entre la peau et le périoste du genou et/ou la synovie et le périoste du genou.

2. Dispositif selon la revendication 1, comprenant en outre :
a) un moyen pour limiter le mouvement de la synovie du genou, dans lequel le dispositif fixe la synovie du genou ; et/ou
b) un moyen pour comprimer la synovie du genou, dans lequel le dispositif positionne et contraint le moyen de compression sur la synovie du genou.

3. Dispositif selon la revendication 1, comprenant en outre :
a) un moyen pour comprimer les deux côtés latéraux du genou au-dessus de l'articulation du genou dans la région de la métaphyse fémorale distale, dans lequel le dispositif positionne et contraint le moyen de compression sur les deux côtés latéraux du genou au-dessus de l'articulation de genou dans la région de la métaphyse fémorale distale ; et/ou
b) un moyen pour comprimer les deux côtés latéraux du genou au-dessus de l'articulation du genou dans la région de la métaphyse tibiale et fibulaire proximale, dans lequel le dispositif positionne et contraint le moyen de compression des deux côtés latéraux du genou au-dessous de l'articulation de genou dans la région de la métaphyse tibiale et fibulaire proximale.

4. Dispositif selon la revendication 1, comprenant en outre :
un moyen pour limiter le mouvement de l'articulation de genou, dans lequel le dispositif fixe et immobilise l'articulation de genou à un angle fixe dans la plage de 130° à 150°.

5. Dispositif selon les revendications 1 à 4, dans lequel :
a) le moyen pour comprimer à partir des deux côtés latéraux est actionné avec une force identique sur les deux côtés latéraux ; et/ou
b) la force du moyen de compression est ajustée par une ou plusieurs vis ou boutons ; et/ou
c) le moyen de compression se compose de patins concaves ; et/ou
d) le moyen de compression se compose d'un matériau qui est facile à nettoyer.

6. Dispositif selon les revendications 1 à 5, dans lequel :
le dispositif est dépourvu de partie supérieure, de sorte que la totalité ou une partie de la peau entourant la rotule et/ou la totalité ou une partie de la peau au-dessus de la rotule et/ou la totalité ou une partie de la peau au-dessous de la rotule n'est pas recouverte par le dispositif.

7. Dispositif selon les revendications 1 à 6, dans lequel :
a) le dispositif est fermement fixé à une surface solide, telle qu'un brancard, un lit d'hôpital ou un canapé lit, dans lequel un raccordement rigide entre le dispositif et la surface solide persiste ; et/ou
b) le dispositif est fermement fixé à un dispositif médical à ultrasons concentrés à haute intensité utilisé pour l'ablation thermique, dans lequel un raccordement rigide entre le dispositif et le dispositif médical à ultrasons concentrés à haute intensité persiste.
